# EUROPEAN PATENT APPLICATION

(11) **EP 1 741 391 A2**
(43) Date of publication of application: **10.01.2007**
(21) Application number: 06253495.3
(22) Date of filing: 04.07.2006
(51) Int. Cl.: A61B 17/11

(54) **A method for interconnecting hollow bodies**

(30) Priority: 07.07.2005 US 176818
(71) Applicant: Cordis Corporation, Miami Lakes, FL 33014 (US)
(72) Inventor: Tanaka, Don, Saratoga, CA 95070 (US)
(74) Representative: Belcher, Simon James

(57) **Abstract**

A system for joining together two hollow bodies is provided. The system includes a magnetic frame that is placed around a severed end of a hollow body. A positioning member is located and fixed within the lumen of a hollow body. The frame is mounted on a first substantially tubular hollow member that slides along the length of the positioning member. A coaxial space exists between the positioning member and the hollow member. The first member is positioned such that the outer wall of the hollow body is located within the coaxial space. A second substantially tubular hollow member is mounted around the outside of the first member and slid there along until it contacts an end of the frame.. As the second member is slid relative to the first member, the frame is displaced around the outer wall of the hollow body.

## Description

This invention generally relates to devices that are used to interconnect hollow bodies. In particular, this invention relates to a device constructed from super elastic material that magnetically couples the ends of two hollow bodies maintaining fluid contact there between.

In the human body there are numerous hollow members, for example blood vessels and ducts that carry necessary fluids to internal organs or allow for the excretion of those fluids. The human body is often subject to trauma or other injury that may cause the hollow members therein to become severed or otherwise damaged. In such a case it is necessary to repair the damage to the hollow member by attaching the severed ends of the hollow members together. This must be accomplished in a manner such that the two ends of the hollow member are in sealed fluid communication. Adverse consequences may result from an improperly joined hollow member. For example, a blood vessel that is not properly anastomosed may leak, create thrombus, and/or lead to stenosis at the connection site possibly requiring further surgery and increasing the risk of stroke

The current preferred standard for joining hollow bodies together is by means of suturing. This method presents numerous limitations. Suturing can be time consuming because placement, suture tightness and stitch size must be precisely gauged. Surgeons must delicately sew the hollow bodies together being careful not to suture too tightly so as to block the lumen of the hollow bodies or tear the delicate tissue. Conversely, the hollow bodies may be sewn too loosely or may be improperly placed causing a faulty seal to be formed and fluid to leak. The loss of a bodily fluid presents adverse consequences for the patient. For example, the loss of blood results in deleterious effects on the patient's hemodynamics that may endanger the patient's life.

In order to overcome the dangers and disadvantages associated with suturing, various instruments for joining hollow bodies together have been developed. US-A-2003/0088256 ―Conston, discloses an implantable device for interconnecting human vessels. The device comprises at least two pair of flexible support members extending angularly from opposite sides of the top and bottom portion of a tubular connector. The supports are placed within the opening of the vessels being anastamosed. The supports impinge on the inner walls of the vessels such that the two vessels are brought into sealing contact with the tubular connector. The connector acts as a conduit between the two vessels.

Conton provides a configuration for joining vessels in a side-to-to side manner. It is often necessary, however, to join hollow bodies in an end-to-end manner. Conton relies upon the support members to impinge on the walls of the vessels. Employing Conton to join two vessels end-to-end would require the supports to impinge on the sidewalls of the vessel in a substantially perpendicular manner to the longitudinal axis of the vessel in order to provide the necessary sealing force. This could damage the intima of the vessel, result in puncture, or cause the sidewalls of the vessel to bulge such that a tight seal is not maintained.

Yet another instrument is disclosed in US-6,352,543 - Cole. Cole discloses a device and methods for forming an anastomosis between hollow bodies using magnetic force. The device comprises two generally ring shaped securing components that have a magnetic field producing member contained therein. In forming an end-to-end anastomosis, the ends of the hollow bodies to be joined are passed through openings in the securing members. The ends are then folded over the securing members. The magnetic securing members are brought into proximity such that the magnetic force holds the two sections together creating an anastomosis between the hollow bodies.

Cole additionally discloses a system for delivering the securing members within the openings of the hollow bodies to be joined. The system comprises a base that receives and locks the ring shaped securing member thereto. The delivery device is positioned within an opening in a hollow body and unlocked, placing the securing member into the desired position.

The delivery system of Cole is useful in forming a side-by-side anastamosis. This system, however, would not be useful in performing an end-to-end anastamosis since it would interfere with sliding the ends of the hollow bodies into the securing members. Thus, a surgeon would be required to manually form the anastamosis. A manual anastamosis procedure is complicated when the hollow bodies being joined have a small diameter, for example, capillaries. More importantly, the securing members of Cole are constructed from a material that is non-elastic. Even if Cole disclosed a delivery system that could join small diameter hollow bodies in an end-to-end manner, the securing members would not allow precise manipulation of the delivery system.

Currently, there is no apparatus, delivery system or method that can join small diameter hollow bodies together in a precise and repeatable way. The present invention is designed to address this need.

According to the invention, methods for placing a magnetic frame over a hollow body and joining together two hollow bodies are provided. The severed ends of two hollow bodies to be joined together are located. Frames, having magnetic qualities, are placed around the outside of the severed ends of the two hollow bodies. The hollow bodies are brought together and maintained in fluid communication via magnetic force.

In particular, the severed or damaged end of a first hollow body is located. A positioning member is located and fixed within the lumen of a hollow body. A frame is mounted on a first substantially tubular hollow member that is slidable along the length of the positioning member. A coaxial space exists between the positioning member and the hollow member. The first member is positioned such that the outer wall of the hollow body is located within the coaxial space. A second substantially tubular hollow member is slidably mounted around the outside of the first member. The second member is slid along the first member until it contacts the frame. The first and second members are slid to each other disposing the frame around the outer wall of the hollow body. This process is repeated for a second hollow body. Once the frames are in place the two hollow bodies are brought into contact with each other so that the lumens are in fluid communication. The magnetic frames pull the two hollow bodies together and maintain a tight, fluid seal there between.

In order to enhance the tight fluidic seal between two hollow bodies, it is desirable to fold the wall of the hollow body over the frame. In a further embodiment of the invention, the system includes a manipulator for folding the wall of the hollow body over the frame. After the frame is disposed around the outside of the hollow body, the first and second members are slid towards the proximal end of the positioning member where they are removed. The manipulator is placed onto the positioning member and slid towards its proximal end where the manipulator engages the wall of the hollow body. As the manipulator is slid further towards the proximal end of the positioning member it folds the wall of the hollow body over the frame.

The frame is constructed from a superelastic material, for example, Nitinol (Ni-Ti). Use of a super elastic material allows the frame to be deformed and restrained in the deformed condition to facilitate the placement of the device containing the material around the hollow body. The frame may comprise a solid, substantially hollow body. In an alternative embodiment of the invention, the frame is a flexible mesh structure. The mesh structure may be formed from a plurality of individually flexible thread elements defining a helix. The flexible thread elements may comprise wires that are interconnected to form the helix. Alternatively, a solid tube of material is cut to form the flexible thread elements and define the helix.

Part of the frame may be constructed from a magnetic material so long as the desired super elastic properties of the frame are maintained. Alternatively, at least one magnet is disposed at an end of the tubular frame. For example, the magnet may be a single, substantially tubular magnet that fits over the outside of the frame. Alternatively, multiple magnets are disposed at one end of the frame.

The positioning member is a flexible, elongated member that can be placed within the lumen of the hollow body. The positioning member includes an anchoring apparatus to fix the member within the lumen. In one embodiment of the invention, the positioning member comprises a catheter having an inflation lumen running along its length. The anchoring apparatus comprises an inflatable member mounted to the positioning member and in fluid communication with the inflation lumen of the catheter. For example, the anchoring member may comprise a substantially compliant balloon constructed from nylon or mylar.

The first and second substantially tubular hollow members are constructed from a flexible material. The diameter of the first member is greater than that of the positioning member, mounted therein, such that a coaxial space is defined there between. The first member is mounted within the second substantially tubular member. The positioning member, first member and second member are all slidable relative to each other along their lengths. For example, the positioning member is located and fixed within the lumen of the hollow body. Thereafter, the first member is slid over the positioning member, towards the distal end of the hollow body.

The manipulator comprises a substantially tubular body having a plurality of fingers mounted to it. The substantially tubular body is mounted on, and slid toward the distal end of, the positioning member until the fingers engage the inner wall of the hollow body. As the manipulator is slid further towards the distal end of the positioning member, the fingers fold the wall of the hollow body over the frame.

Embodiments of the invention will now be described by way of example with reference to the accompanying drawings, in which:
Figure 1 is a perspective view of the frame/flexible mesh structure of the present invention
Figure 2 is a side view of the hollow body couplinig system of the present invention;
Figure 3 is a view of the hollow body coupling system of the present invention taken along line 3-3 of Figure 2;
Figure 4A is an end view of a hollow body having a severed end;
Figure 4B is a side cutaway view showing the lumen of a hollow body taken along line B-B of Figure 4A;
Figure 5 is a side cutaway view showing a positioning member inserted into the lumen of the hollow body of Figure 4B;
Figure 6 is a side cutaway view showing the positioning member fixed with the lumen of the hollow body of Figure 5;
Figure 7 is a side cutaway view showing the first and second hollow members mounted on the positioning member and placed over the outer wall of the hollow body of Figure 6;
Figure 8 is a side cutaway view showing the frame being disposed over the outer wall of the hollow body of Figure 7;
Figure 9 is a side cutaway view showing the frame disposed around the outer wall of the hollow body of Figure 8;
Figure 9A is a side view of a hollow body wall manipulator;
Figure 9B is a view of the hollow body wall manipulator taken along line B-B of Figure 9A;
Figure 10 is a side cutaway or sagittal view showing the hollow body wall manipulator disposed over the positioning member;
Figure 11 is a side cutaway view showing the manipulator engaging the inner wall of the hollow body;
Figure 12 is a side cutaway view showing the manipulator further engaging the inner wall of the hollow body;
Figure 13 is a side cutaway view showing the wall of the hollow body folder over the frame;
Figure 14 is a side cutaway view showing the hollow body with the frame mounted thereto.

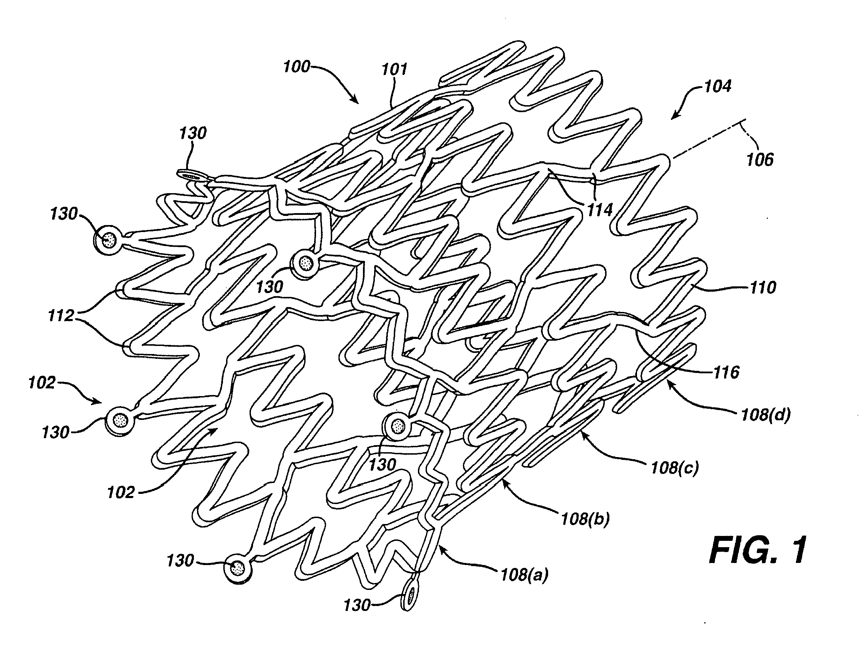

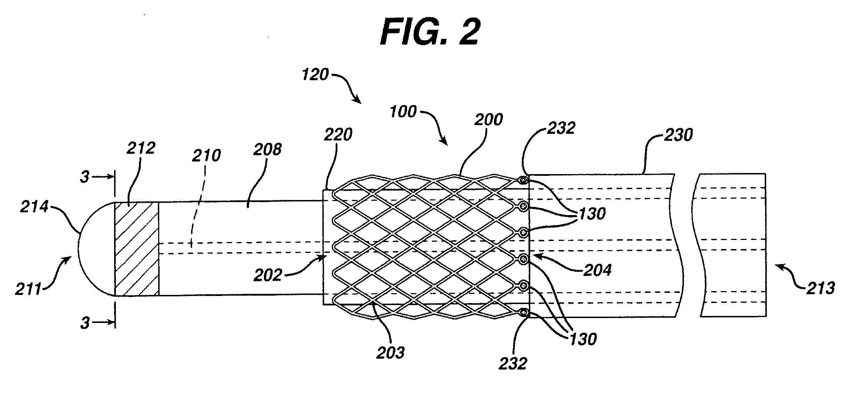

An apparatus, system and method for joining together two hollow bodies in fluid communication will be described with reference to Figures 1-14. As shown in Figures 1 and 2, the apparatus generally comprises a frame 100 having at least one magnet 130 mounted thereon. The frame 100 is disposed over the outer wall of a hollow body 10. A second frame, not shown, is disposed over the outer wall of a second hollow body, also not shown. When the hollow bodies 10 are placed into contact the magnetic frames link the two bodies 10 together in fluidic communication.

The frame 100 is constructed from a super elastic material. One example of such super elastic material is Nitinol (Ni-Ti). Use of super elastic materials allows the frame 100 to be restrained in a deformed condition to facilitate the placement of the frame 100 containing the material around the hollow body 10. For example, the super elastic characteristics allow the frame 100 to have a first, expanded diameter for mounting to the end of a sheath or other apparatus 220 used to position the frame 100 as shown in Figure 2 around the outside of the hollow body 10. When the frame 100 is slid off of the sheath it resumes a second, smaller diameter allowing for disposal over the outer wall 12 of the hollow body 10.

The frame 100 is substantially tubular and may comprise a solid, substantially hollow body. In some applications, however, it is desirable for the frame 100 to exhibit greater flexibility in which case the frame comprises a flexible mesh structure 200, shown in Figure 2. The mesh structure 200 may be formed from a plurality of individually flexible thread elements 203 defining a helix. Alternatively, a solid tube of material may be cut to form the flexible thread elements 203.

In another embodiment, shown in Figure 1, frame 100 is a substantially tubular member 101 having front and back open ends 102 and 104 and a longitudinal axis 106 extending there between. The substantially tubular member 101 is made from a plurality of adjacent hoops 108, Figure 1 showing hoops 108(a) - 108(d), extending between the front and back ends 102 and 104. The hoops 108 include a plurality of longitudinal struts 110 and a plurality of loops 112 connecting adjacent struts, wherein adjacent struts are connected at opposite ends so as to form a substantially S or Z shape pattern. The loops 112 are curved, substantially semi-circular with symmetrical sections about their centers 114.

Member 101 further includes a plurality of bridges 116 which connect adjacent hoops 108. Each bridge 116 has one end attached to one strut and/or loop, and another end attached to a strut and/or loop on an adjacent hoop. The bridges 116 connect adjacent struts together at bridge to loop connection points 114. The bridge to loop connection points 114 are separated angularly with respect to the longitudinal axis. That is, the connection points 114 are not immediately opposite each other. Essentially, one could not draw a straight line between the connection points 114 wherein such line would be parallel to the longitudinal axis of the tubular member 101. The geometry described above helps to better distribute strain throughout the tubular member 101 and prevents metal-to-metal contact when the tubular member 101 is bent. The number and nature of the design of the struts 110, loops 112 and bridges 116 are important factors when determining the working properties and fatigue life properties of the tubular member 101.

Part of the frame 100 may be constructed from a magnetic material so long as the desired super elastic properties of the frame 100 are maintained. Alternatively, at least one magnet 130 is disposed at an end of the frame. The magnet may be a single, substantially tubular magnet that fits over the outside of the frame, not shown in the Figures. Alternatively, multiple magnets 130 are disposed at even intervals around one end of the frame 100, Figure 1.

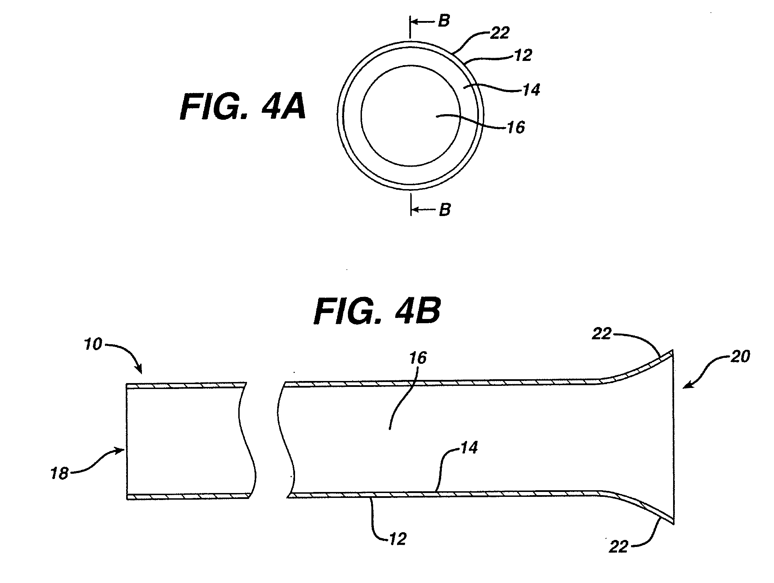

A system 120 for connecting two hollow bodies together is described with reference to Figures 2-14. The hollow body, shown in Figures 4A and 4B, is a substantially tubular hollow member having a lumen 16 located therein. The hollow body 10 has an outer wall 12 and an inner wall 14 and a distal 8 and a proximal end 20. As shown in Figures 4A and 4B, the proximal end 20 of the hollow body 10 terminates in a severed or damaged section 22.

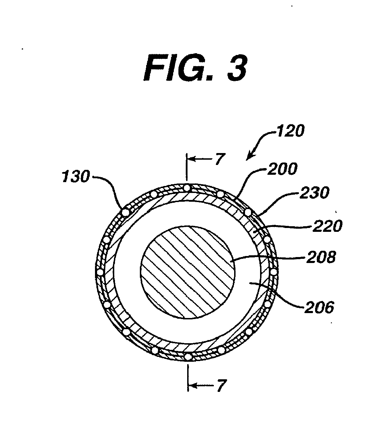

As shown in Figures 2 and 3, the system 120 includes frame 100, described above, that is placed around the severed end 22 of a hollow body 10. The system further includes a positioning member 208, a first hollow member 220 and a second hollow member 230. The frame 100 is mounted on the outer wall of the first hollow member 220. The positioning member 208 is mounted within the first hollow member 220 such that member 208 and hollow member 220 may be slid in a longitudinal direction relative to each other. The diameter of the first hollow member 220 is larger than the positioning member 208 such that an interstitial or coaxial space 206 is defined there between. The first hollow member 220 is mounted within the second hollow member 230 so that each may be slid relative to each other in a longitudinal direction. As shown in Figure 2, the second hollow member 230 is advanced along the first hollow member 220 until the leading edge 232 of the second member abuts the proximal end 204 of the frame 100.

The positioning member 208 is a flexible, elongated shaft that can be placed within the lumen 16 of the hollow body 10. The positioning member 208 preferably has a rounded tip 214 to allow for navigation within the lumen 16 without damaging the inner wall 14. In addition, positioning member 208 includes an anchoring apparatus 212 to fix the member 208 within the lumen 16. In one embodiment of the invention, the positioning member 208 comprises a catheter having an inflation lumen 210 running along its length. The inflation lumen 210 is in communication with a fluid source, not shown, located at its proximal end 213. The anchoring apparatus 212 comprises an inflatable member mounted to the positioning member 208 and in fluid communication with the inflation lumen 212 of the catheter. For example, the anchor 212 may comprise a substantially compliant balloon constructed from mylar, nylon, or Nitinol.

Alternatively, the anchor 212 may comprise fingers constructed from Nitinol that expand when introduced into the lumen 16 locking in place positioning member 208. Nitinol exhibits shape memory characteristics that allow the fingers to have a memorized, extended position. Shape memory characteristics are imparted to the alloy by heating the metal at a temperature above which the transformation from the martensite phase to the austenite phase is complete, i.e. a temperature above which the austenite phase is stable (the Af temperature). The shape of the metal during this heat treatment is the shape "remembered." The heat-treated metal is cooled to a temperature at which the martensite phase is stable, causing the austenite phase to transform to the martensite phase. The metal in the martensite phase is then plastically deformed, e.g. to facilitate the entry thereof into the lumen 16. Subsequent heating of the deformed martensite phase to a temperature above the martensite to austenite transformation temperature causes the deformed martensite phase to transform to the austenite phase, and during this phase transformation the metal reverts back to its original shape if unrestrained.

The first hollow member 220 is preferably constructed from a flexible material. The frame 100, having a first, expanded diameter, is mounted to the distal end of the first hollow member 220, Figure 2. In one embodiment of the invention, the first hollow member 220 comprises a polymeric sheath having sufficient rigidity to support the frame 100 when it is in the expanded condition without collapsing member 220. The hollow body 10 resides within the space 206 when the first member 220 is slid towards the distal end 18 of the hollow body 10. This places the frame 100 into position for disposition around the outer wall 12 of hollow body 10.

The second hollow member 230 may be constructed form the same material as the first hollow member 220. Alternatively, the second hollow member 230 may be constructed from a more rigid material that can exert adequate pushing force on frame 100 without exhibiting any deformation. In any even, the materials used to construct the first 220 and second 230 hollow members should permit sliding engagement there between. The second hollow member 230 has an outer diameter larger than the outer diameter of the first hollow member 220 forming a ridge 132. When the second hollow member 230 is slid towards the tip 214 of the positioning member, ridge 132 impinges on the proximal end 204 of frame 100.

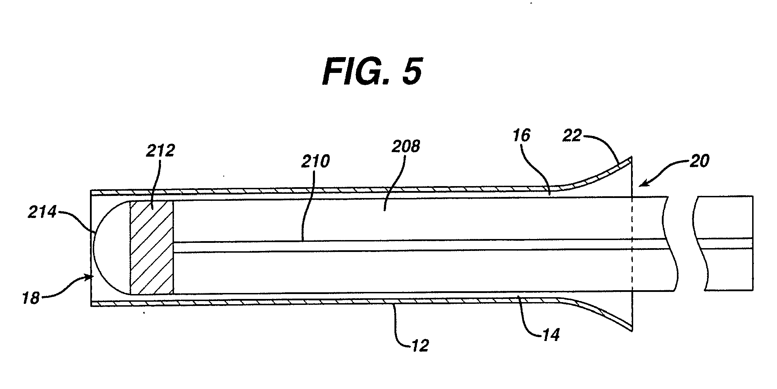

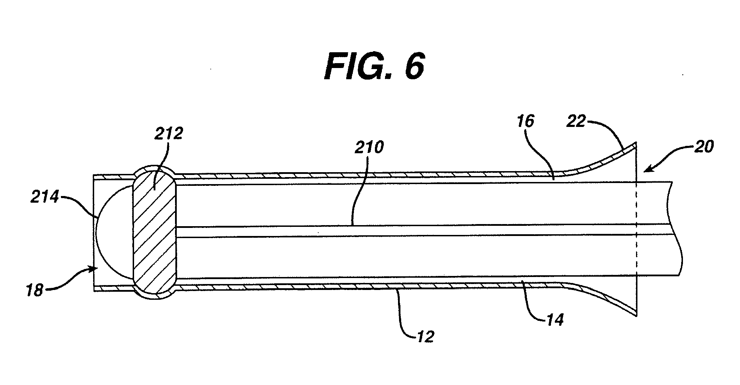

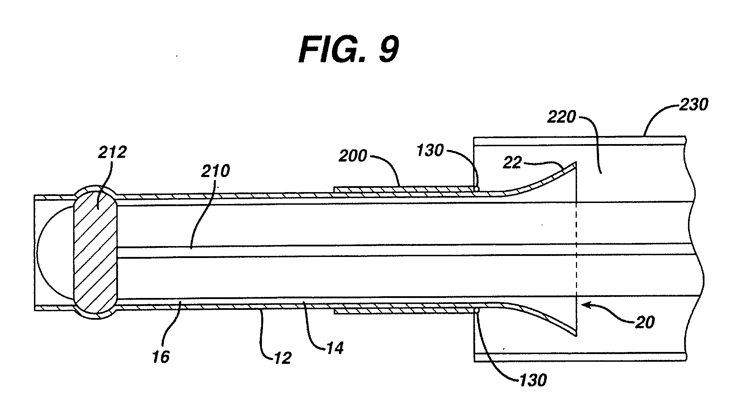

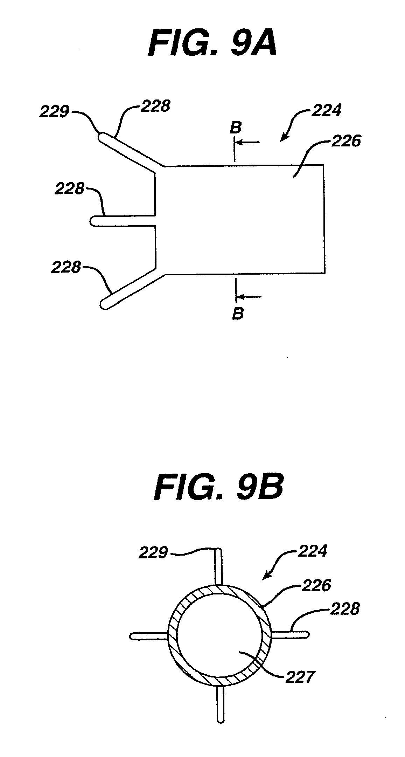

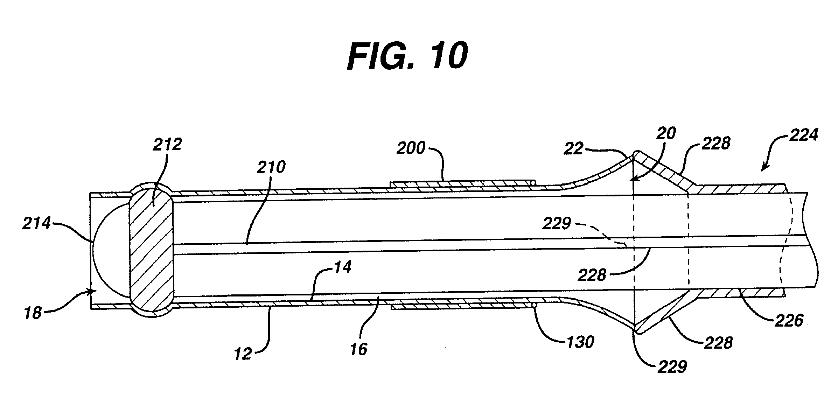

A manipulator 224 for folding the wall of the hollow body 10 over the frame 100 is shown in Figures 9A and 9B. The manipulator 224 comprises a substantially tubular body 226 constructed from a flexible material. The substantially tubular body 226 defines an opening 227 having an inner diameter that is at least that of the outer diameter of positioning member 208. Angularly oriented fingers 228 are mounted on the substantially tubular body 226 and preferably include oval or rounded edges 229 to prevent any damage to the inner wall 14 of the hollow body 10. The substantially tubular body 226 is mounted on the positioning member 208. As the substantially tubular body 226 is slid toward the tip 214 of the positioning member 208 the edges 229 of the angularly oriented fingers 228 engage the inner wall 14 of the hollow body 10, Figure 10.
The system described above is utilized to join two or more hollow bodies in fluid communication. In operation, the severed end 22 of a hollow body 10 is located. A positioning member is placed within the lumen 16 of the hollow body 10, Figure 5. Once located within the lumen 16, the positioning member is fixed within the lumen 16 by the anchoring apparatus 212, Figure 6.

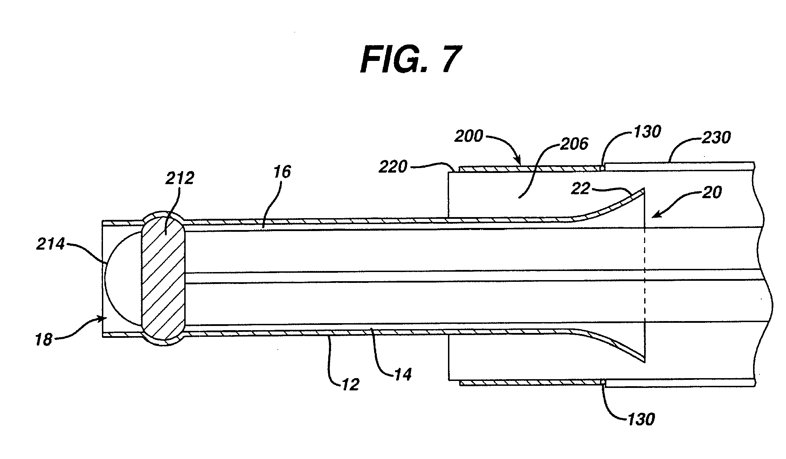

A super elastic frame 100 is deformed to have a first expanded diameter and is mounted on a first substantially tubular hollow member 220, Figure 2. The first member is slid along the positioning member 208 towards tip 214 until the outer wall 12 of the hollow body 10 is located within the coaxial space 206 and the frame is positioned behind the severed end 22 of hollow body 10, Figure 7. A second substantially tubular hollow member 230 is slidably mounted around the outside of the first member 220. The second member 230 is slid along the first member 220 until it contacts the proximal end 204 of the frame 100, Figure 7.

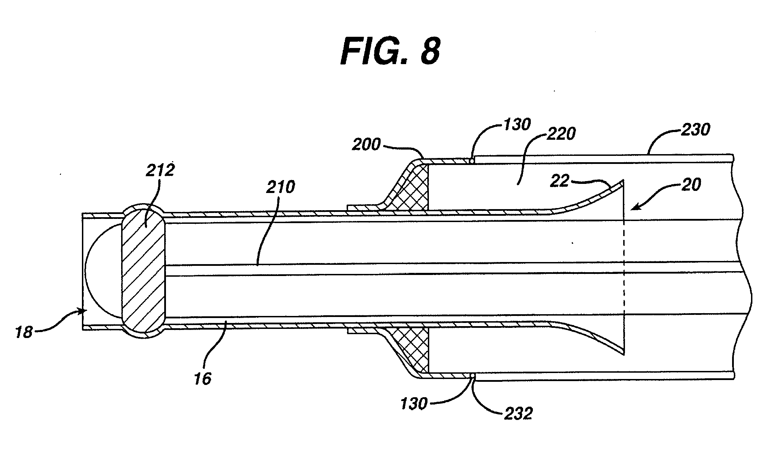

The first 220 and second 230 members are slid relative to each other such that the frame 100 is pushed or pulled off of the distal end of first member 220 where it assumes a second, smaller diameter, Figure 8. Frame 100 is then disposed over the outer wall 12 of hollow body 10. Thereafter, the positioning 208, first 220 and second 230 members are removed from the hollow body 10. The process is repeated for a second hollow body. Once the frames are in place the two hollow bodies are brought into contact with each other so that the lumens are in fluid communication. The frames, having magnets mounted thereon, or being magnetic themselves, pull the two hollow bodies together and maintain a tight, fluid seal there between.

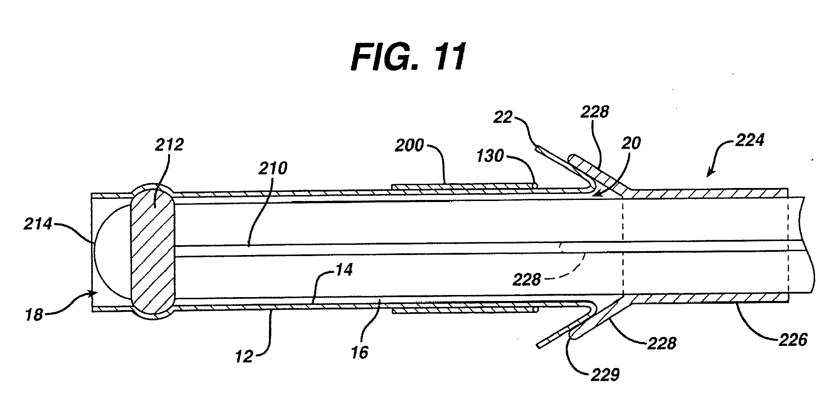

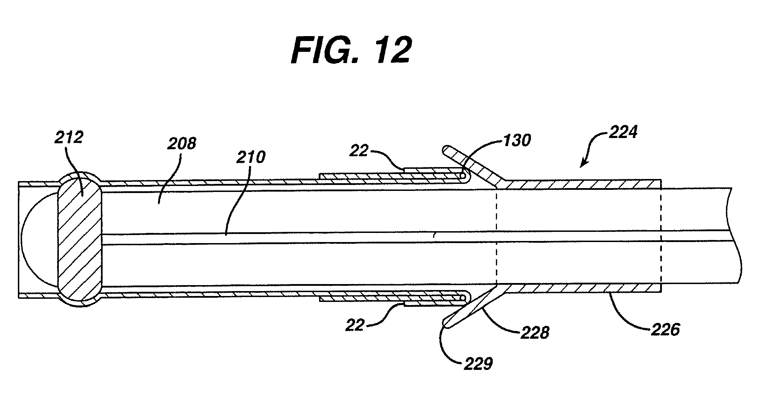

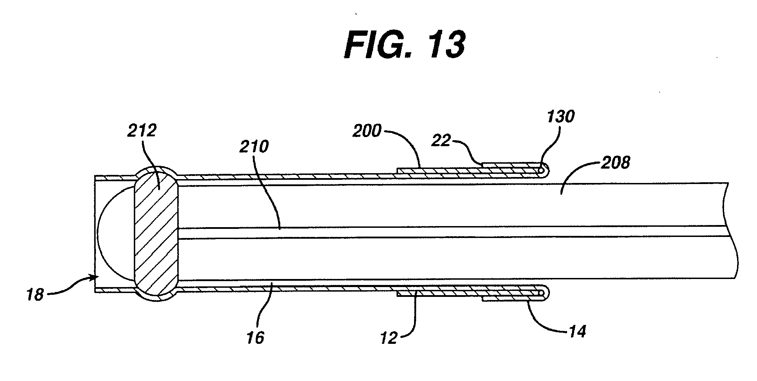

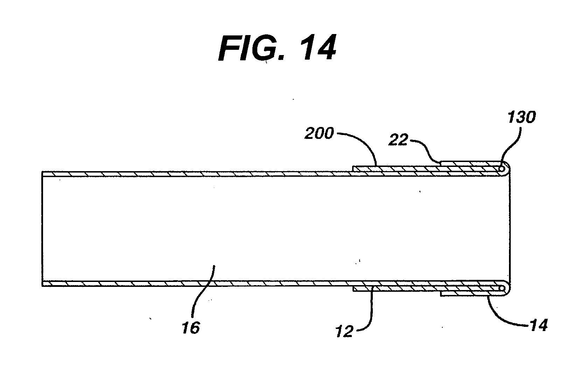

In order to enhance the tight fluidic seal between two hollow bodies and ensure that the inner walls 14 of the hollow bodies 10 contact each other, it is desirable to fold the wall of the hollow body 10 over the frame 100 prior to joining the two hollow bodies. After placement of the frame 100 around the outside of hollow body 10, the first and second hollow members are removed by sliding the members towards the distal end 213 of the positioning member 208. The manipulator 224 is placed over the positioning member 208 and slid towards tip 214. Fingers 228 engage the inner wall 14 of hollow body 10, Figure 10. As the manipulator 224 is slid further towards the tip 214 fingers 228 bend the wall of hollow body of the positioning member it folds the wall of the hollow body over the frame 100, Figures 11 and 12. Once the wall of the hollow body 10 is folded over the frame 100, the manipulator 224 and then the positioning member208 are removed, Figures 13 and 14. The process is repeated for another hollow body and the two hollow bodies are brought into contact with each other so that the lumens are in fluid communication. The frames, having magnets mounted thereon, or being magnetic themselves, pull the two hollow bodies together and maintain a tight, fluid seal there between.

## Claims

1. A method for joining at least two hollow bodies comprising the steps of:
a) locating the ends of the two hollow bodies to be joined;
b) inserting a first flexible elongated body into a lumen of one of the at least two hollow bodies;
c) fixing a distal end of the first elongated body within the lumen of the one of the at least two hollow bodies;
d) mounting a substantially hollow, tubular frame having at least one magnet mounted thereon over a second hollow elongated body;
e) sliding the second elongated body over the first flexible elongated body so that an outer wall of one of the at least two hollow bodies is located between the first flexible elongated body and the second flexible elongated hollow body;
f) sliding a third hollow elongated body along an outer wall of the second hollow elongated body until a leading edge of the third elongated hollow body impinges upon a side of the frame having the magnets mounted thereon;
g) sliding the second and third hollow elongated bodies relative to each other so as to move the frame off of the second elongated hollow body and onto the outer wall of one of that at least two hollow bodies;
h) removing the second and third elongated hollow bodies;
i) folding one of the at least two hollow bodies over the frame;
j) repeating steps a)-i) for another of the at least two hollow bodies; and
k) bringing the at least two hollow bodies in proximity such that the magnets couple the at least two hollow bodies together and form a tight seal therebetween.

2. A method for placing a frame over a hollow body to be joined to another hollow body comprising the steps of:
a) locating an end of the hollow body;
b) inserting a first flexible elongated body into a lumen of the hollow body;
c) fixing a distal end of the first flexible elongated body within the lumen;
d) mounting a substantially hollow, tubular frame having at least one magnet mounted thereon over a second hollow elongated body;
e) sliding the second hollow elongated body over the first flexible elongated body so that an outer wall of one of the at least two hollow bodies is located between the first flexible elongated body and the second flexible elongated hollow body;
f) sliding a third hollow elongated body along an outer wall of the second hollow elongated body until a leading edge of the third elongated hollow body impinges upon a side of the frame having the magnets mounted thereon; and
g) sliding the second and third hollow elongated bodies relative to each other so as to move the frame off of the second elongated hollow body and onto the outer wall of the hollow body.

3. The method of claim 1 or 2 wherein the first flexible elongated body comprises a catheter having a balloon located at a distal end of the catheter, said catheter being fixed within the lumen of one of the at least two hollow bodies by inflating the balloon.

4. The method of claim 1 or 2 wherein the substantially hollow, tubular frame comprises a plurality of hoops wherein each adjacent hoop is joined together by a plurality of bridges.

5. The method of claim 4 wherein the plurality of hoops comprise a plurality of struts wherein each adjacent strut is connected together by a plurality of loops.

6. The method of claim 2 further comprising the step of folding one of the at least two hollow bodies over the frame by sliding a manipulator over the first flexible elongated body and moving said manipulator towards the distal end of the first flexible elongated body until it contacts the inner wall of one of the at least two hollow bodies and bends it over a proximal end of the frame.

7. The method of claim 1 wherein folding one of the at least two hollow bodies over the frame is accomplished by sliding a manipulator over the first flexible elongated body and moving said manipulator towards the distal end of the first flexible elongated body until it contacts the inner wall of one of the at least two hollow bodies and bends it over a proximal end of the frame.

8. The method of claim 6 or 7 wherein the manipulator has at least two fingers mounted thereon and oriented at an angle to a longitudinal axis of the first flexible elongated body
